# EUROPÄISCHE PATENTSCHRIFT

(11) **EP 1 199 369 B1**
(45) Veröffentlichungstag und Bekanntmachung des Hinweises auf die Patenterteilung: **13.02.2008**
(21) Anmeldenummer: 01123744.3
(22) Anmeldetag: 04.10.2001
(51) Int. Cl.: C12P 41/00, C12N 9/90

(54) **Verwendung der Acetylaminosäureracemase aus Amycolatopsis orientalis zur Racemisierung von Carbamoylaminosäuren**
Use of the acetylamino acid racemase from Amycolatopsis orientalis in the racemization of carbamoylamino acids
Utilisation de la racémase d'acides aminés acylés d'Amycolatopsis orientalis pour la racémisation d'acides carbamoylaminés

(30) Priorität: 11.10.2000 DE 10050124
(43) Veröffentlichungstag der Anmeldung: 24.04.2002
(73) Patentinhaber: Evonik Degussa GmbH, 40474 Düsseldorf (DE)
(72) Erfinder: Bommarius, Andreas, Prof., 30327 Atlanta (US); Verseck, Stefan, Dr., 63452 Hanau (DE); Drauz, Karlheinz, Prof., 63579 Freigericht (DE); Kula, Maria-Regina, Prof., 52382 Niederzier (DE)

(56) Entgegenhaltungen:
- EP-A- 0 474 965
- EP-A- 1 074 628
- VERSECK S. ET AL.: "Screening, overexpression and characterization of an N-acyamino acid racemase from Amylocatopsis orientalis subsp. lurida." APPL. MICROBIOL. BIOTECHNOL., Bd. 55, Nr. 3, 13. März 2001 (2001-03-13), Seiten 354-361, XP002202059
- PATENT ABSTRACTS OF JAPAN vol. 2000, no. 19, 5. Juni 2001 (2001-06-05) & JP 2001 046088 A (DEGUSSA HUELS AG), 20. Februar 2001 (2001-02-20)

## Beschreibung

Die vorliegende Erfindung richtet sich auf die Verwendung einer N-Acetylaminosäureracemase (AAR) in einem Verfahren zur Racemisierung von N-Carbamoylaminosäuren.

Optisch reine Aminosäuren sind für die chemische Synthese sowie die parenterale Ernährung wichtige Ausgangsstoffe. Zur Herstellung optisch reiner Aminosäuren sind dem Fachmann viele Möglichkeiten bekannt. U.a. bieten sich diesbezüglich enzymatische Verfahren an, da sie zum einen katalytisch arbeiten und zum anderen die Aminosäuren mit sehr hohen Enantiomerenanreicherungen herzustellen gestatten.

Ein bekanntes enzymatisches Verfahren geht dabei von racemischen Hydantoinen aus, welche mittels Hydantoinasen in N-carbamoylgeschützte Aminosäuren transformiert werden. Anschließend werden diese durch Carbamoylasen in die Aminosäuren umgesetzt.
Vorzugsweise erfolgt die Trennung der in dieser Reaktionssequenz auftretenden Racemate auf der Basis der N-carbamoylgeschützten Aminosäuren, da sowohl L- als auch D-selektive Carbamoylasen zur Verfügung stehen (Park et al., Biotechnol. Prog. 2000, 16, 564-570; May et al., Nat Biotechnol. 2000, 18, 317-20; Pietzsch et al., J. Chromatogr. B Biomed. Sci. Appl. 2000, 737, 179-86; Chao et al., Biotechnol. Prog. 1999, 15, 603-7; Wilms et al., J. Biotechnol. 1999, 68, 101-13; Batisse et al., Appl. Environ. Microbiol. 1997, 63, 763-6; Buson et al., FEMS Microbiol. Lett. 1996, 145, 55-62).
Um einen vollständigen Umsatz der eingesetzten Hydantoine in optisch reine Aminosäuren zu gewährleisten, geschieht die dazu notwendige Racemisierung bisher auf der Basis der Hydantoine in chemischer oder enzymatischer Art und Weise (EP 745678; EP 542098; Schema 1).

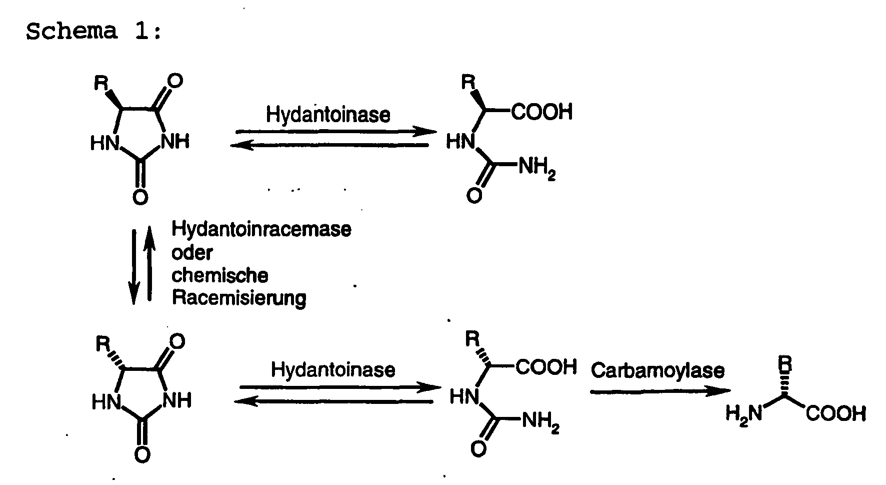

Aus Streptomyces atratus Y-53 (Tokuyama et al., Appl. Microbiol. Biotechnol. 1994, 40, 835-840) und Amycolatopis sp. TS-1-60 (Tokuyama et al., Appl. Microbiol. Biotechnol. 1995a, 42, 853-859) sowie Amycolatopsis orientalis sp. lurida (DE19935268) sind N-Acetylaminosäureracemasen (AARs) bekannt. Von der TS-1-60 ist eine allerdings sehr geringe Aktivität bei N-carbamoylgeschützten Aminosäuren feststellbar. Darüberhinaus besitzt dieses Enzym den Nachteil einer sehr hohen Metallionenabhängigkeit, was für den Einsatz dieses Enzyms in einem großtechnischen Prozeß nachteilig erscheint.

Aufgabe der vorliegenden Erfindung war es deshalb, die Verwendung einer N-Acetylaminosäureracemase zur gegenüber dem Stand der Technik verbesserten Racemisierung von N-Carbamoylaminosäuren anzugeben. Diese Racemase sollte in einem Verfahren zur Herstellung von optisch reinen Aminosäure ausgehend von racemischen Hydantoinen großtechnisch vorteilhaft einsetzbar sein.

Gelöst wird diese Aufgabe durch die Verwendung der AAR gemäß Anspruch 1. Ansprüche 2 und 3 richten sich auf bevorzugte Ausgestaltungen des erfindungsgemäßen Racemisierungsverfahrens.

Dadurch, daß man in einem Verfahren zur Racemisierung von N-Carbamoylaminosäuren eine N-Acetylaminosäureracemasen (AAR) aus Amycolatopsis orientalis subspecies lurida (Seq. 2) verwendet, erhält man aufgrund der überraschend erhöhten Aktivität der erfindungsgemäß eingesetzten AAR gegenüber der TS-1-60 im Hinblick auf die Racemisierung von N-Carbamoylaminosäuren die Möglichkeit, in einem verbesserten Verfahren die Äquilibrierung optischer Antipoden von N-carbamoylgeschützten Aminosäuren zu vollziehen.

Dies ist vor dem Hintergund besonders vorteilhaft, daß man somit einen weiteren enzymatischen Schritt in einem Verfahren zur Herstellung von optisch reinen Aminosäuren etablieren kann, welches von Hydantoinen ausgeht (Schema 2). 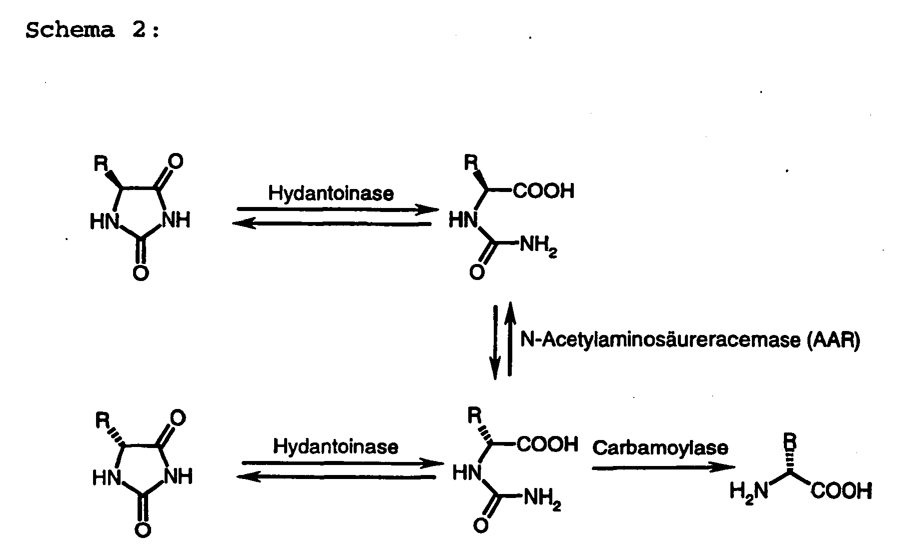 Gegenüber den literaturbekannten enzymatischen Verfahren, welche über eine enzymatische oder ggf. stressende chemische Racemisierung der Hydantoine prozessieren (Schema 1), hat man somit eine weitere vorteilhafte Möglichkeit geschaffen, aus racemischen Hydantoinen optisch reine Aminosäuren zu generieren.

Vorzugsweise wird für das Racemisierungsverfahren die rekombinant hergestellte Variante der AAR aus Amycolatopsis o. sp. lurida gemäß DE19935268 eingesetzt. Aus der DE19935268 ist bekannt, daß diese eine verhältnismäßig geringe Schwermetallionenabhängigkeit (insbesondere in Bezug auf Kobaltionen) und geringe Aminosäureinhibierung aufweist. Dort wird auch deren Generierung als rekombinantes Enzym erläutert.

Das erfindungsgemäße Verfahren wird wie gesagt in einem Gesamtprozeß zur Herstellung von enantiomer angereicherten Aminosäuren oder deren Derivaten ausgehend von Hydantoinen oder N-Carbamoylaminosäuren vorteilhaft eingesetzt. Man geht dabei im Falle der Hydantoine bevorzugt so vor, daß man racemische Hydantoine mittels Hydantoinasen in die korrespondierenden racemischen N-Carbamoylaminosäuren spaltet und diese anschließend durch L- oder D-spezifische Carbamoylasen in die optisch aktiven L- oder D-Aminosäuren umwandelt. Damit sich nicht die nicht umgesetzte optische Antipode einer N-Carbamoylaminosäure im Reaktionsgemisch anreichert, setzt man die optischen Antipoden der N-Carbamoylaminosäuren durch Zugabe der AAR erfindungsgemäß ins Gleichgewicht und kann somit ebenfalls das racemische Hydantoin zur Gänze in optisch reine Aminosäuren umwandeln.

Vorzugsweise erfolgt dieser Prozeß in einem Enzym-Membran-Reaktor (DE 199 10 691.6).

Die genannten Enzyme können in freier Form als homogen aufgereinigte Verbindungen oder als rekombinant hergestellte Enzyme zusammen oder nacheinander verwendet werden. Weiterhin können die Enzyme auch als Bestandteil eines Gastorganismus (Ganzzellkatalysator wie in US09/407062) eingesetzt werden oder in Verbindung mit der aufgeschlossenen Zellmasse des Wirtsorganismus. Möglich ist ebenfalls die Verwendung der Enzyme in immobilisierter Form (Bhavender P. Sharma, Lorraine F. Bailey and Ralph A. Messing, "Immobilisierte Biomaterialiern - Techniken und Anwendungen", Angew. Chem. 1982, 94, 836-852). Vorteilhafterweise erfolgt die Immobilisierung durch Lyophilisation (Dordick et al. J. Am. Chem. Soc. 194, 116, 5009-5010; Okahata et al. Tetrahedron Lett. 1997, 38, 1971-1974; Adlercreutz et al. Biocatalysis 1992, 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycol-monocetylether) (Goto et al. Biotechnol. Techniques 1997, 11, 375-378.

Der Mikroorganismus Amycolatopsis orientalis subsp. lurida ist unter der Nummer DSM43134 bei der Deutschen Sammlung für Mikroorganismen hinterlegt.

Unter AAR wird im Rahmen der Erfindung sowohl das native wie auch das rekombinant hergestellte Enzym verstanden.

Der Begriff enantiomer angereichert bezeichnet das vorliegen einer optischen Antipode im Gemisch mit der anderen in >50%.

Unter Aminosäure wird im Rahmen der Erfindung eine natürliche oder unnatürliche α-Aminosäure verstanden, d.h. daß der am α-C-Atom der α-Aminosäure befindliche Rest sich von einer natürlichen Aminosäure, wie in Beyer-Walter, Lehrbuch der organischen Chemie, S. Hirzel Verlag Stuttgart, 22. Auflage, 1991, S.822f. dargestellt, ableiten kann oder darüberhinaus auch von entsprechenden α-Resten unnatürlicher α-Aminosäuren, welche z.B. in DE19903268.8 aufgeführt sind.

### SEQUENZPROTOICOLL

<110> Degussa-Hüls AG
<120> Verwendung einer Acetylaminosäureracemase zur Racemisierung von Carbamoylaminosäuren
<130> 000337 AM
<140>
   <141>
<160> 2
<170> PatentIn Ver. 2.1
<210> 1
   <211> 1107
   <212> DNA
   <213> Amycolatopsis orientalis
<220>
   <221> CDS
   <222> (1)..(1107)
<400> 1
   gtg aaa ctc agc ggt gtg gaa ctg cgc cgg gtc cgg atg ccg ctc gtg    48
   Val Lys Leu Ser Gly Val Glu Leu Arg Arg Val Arg Met Pro Leu Val
    1               5                  10                  15
   gcc ccg ttc cgg acg tcg ttc ggg acg cag tcc gag cgg gaa ttg ctg    96
   Ala Pro Phe Arg Thr Ser Phe Gly Thr Gln Ser Glu Arg Glu Leu Leu
               20                  25                  30
   ctg gtc cgc gcg gtg acc ccg gcg ggc gag ggc tgg ggc gaa tgt gtc    144
   Leu Val Arg Ala Val Thr Pro Ala Gly Glu Gly Trp Gly Glu Cys Val
           35                  40                  45
   gcg atg gag gcg ccg ctc tac tcg tcg gag tac aac gac gcc gcc gag    192
   Ala Met Glu Ala Pro Leu Tyr Ser Ser Glu Tyr Asn Asp Ala Ala Glu
       50                  55                  60
   cac gtg ctg cgg aac cat ctg atc ccc gca ctg ctg gcg gcc gag gac    240
   His Val Leu Arg Asn His Leu Ile Pro Ala Leu Leu Ala Ala Glu Asp
   65                  70                  75                  80
   gtg acc gcg cac aag gtg acg ccg ttg ctg gcg aag ttc aag ggc cac    288
   Val Thr Ala His Lys Val Thr Pro Leu Leu Ala Lys Phe Lys Gly His
                   85                  90                  95
   cgg atg gcg aag ggc gcg ctg gag atg gcg gtc ctc gac gcc gaa ctc    336
   Arg Met Ala Lys Gly Ala Leu Glu Met Ala Val Leu Asp Ala Glu Leu
               100                 105                 110
   cgc gcg cat gac cgg tcc ttc gcg gcc gag ctg ggg tcc act cgc gac    384
   Arg Ala His Asp Arg Ser Phe Ala Ala Glu Leu Gly Ser Thr Arg Asp
           115                 120                 125
   tcc gtg gcc tgc ggg gtc tcg gtc ggg atc atg gac tcg atc ccg cac    432
   Ser Val Ala Cys Gly Val Ser Val Gly Ile Met Asp Ser Ile Pro His
       130                 135                 140
   ctg ctc gac gtc gtc ggc ggc tac ctc gac gag ggc tac gtc cgg atc      480
   Leu Leu Asp Val Val Gly Gly Tyr Leu Asp Glu Gly Tyr Val Arg Ile
   145                 150                 155                 160
   aag ctg aag atc gag ccc ggc tgg gac gtc gag ccg gtc cgg cag gtg      528
   Lys Leu Lys Ile Glu Pro Gly Trp Asp Val Glu Pro Val Arg Gln Val
                   165                 170                 175
   cgt gag cgc ttc ggt gac gac gtg ctg ctg cag gtc gac gcg aac acc      576
   Arg Glu Arg Phe Gly Asp Asp Val Leu Leu Gln Val Asp Ala Asn Thr
               180                 185                 190
   gcg tac acg ctg ggc gac gcg ccc ctg ctg tcc cgg ctc gac ccg ttc      624
   Ala Tyr Thr Leu Gly Asp Ala Pro Leu Leu Ser Arg Leu Asp Pro Phe
           195                 200                 205
   gac ctg ctg ctg atc gag cag ccg ctc gaa gaa gag gac gtg ctc ggc      672
   Asp Leu Leu Leu Ile Glu Gln Pro Leu Glu Glu Glu Asp Val Leu Gly
       210                 215                 220
   cac gcc gag ctg gcc aag cgg atc cgg acg ccg atc tgc ctc gac gag      720
   His Ala Glu Leu Ala Lys Arg Ile Arg Thr Pro Ile Cys Leu Asp Glu
   225                 230                 235                 240
   tcg atc gtc tcg gcc aag gcc gcc gcg gac gcg atc aag ctc ggc gcc      768
   Ser Ile Val Ser Ala Lys Ala Ala Ala Asp Ala Ile Lys Leu Gly Ala
                   245                 250                 255
   tgc cag atc gtc aac atc aaa ccg ggc cgg gtc ggc gga tac ctc gaa      816
   Cys Gln Ile Val Asn Ile Lys Pro Gly Arg Val Gly Gly Tyr Leu Glu
               260                 265                 270
   gcc cgc cgg gtg cac gac gtc tgc gcg gca cac ggg atc gcg gtg tgg      864
   Ala Arg Arg Val His Asp Val Cys Ala Ala His Gly Ile Ala Val Trp
           275                 280                 285
   tgc ggc ggg atg atc gag acc ggg ctc ggc cgg gcg gcc aac gtc gca      912
   Cys Gly Gly Met Ile Glu Thr Gly Leu Gly Arg Ala Ala Asn Val Ala
       290                 295                 300
   ctg gcc tcg ctg ccc ggc ttc acg ctg ccg ggg gac acc tcg gcg tcc      960
   Leu Ala Ser Leu Pro Gly Phe Thr Leu Pro Gly Asp Thr Ser Ala Ser
   305                 310                 315                 320
   ggc cgg ttc tat cgc acc gac atc acc gag ccg ttc gtg ctg gac gcc     1008
   Gly Arg Phe Tyr Arg Thr Asp Ile Thr Glu Pro Phe Val Leu Asp Ala
                   325                 330                 335
   ggg cat ctg ccg gtg ccg acc ggg ccg ggc ctc ggg gtg act ccg att     1056
   Gly His Leu Pro Val Pro Thr Gly Pro Gly Leu Gly Val Thr Pro Ile
               340                 345                 350
   ccg gat ctt ctg gac gag gtc acc acg gag aaa gcg tgg atc ggt tcg     1104
   Pro Asp Leu Leu Asp Glu Val Thr Thr Glu Lys Ala Trp Ile Gly Ser
           355                 360                 365
   tag 1107
<210> 2
   <211> 368
   <212> PRT
   <213> Amycolatopsis orientalis
<400> 2

### Beispiele:

### Nachweis der Racemaseaktivität des rekombinanten AAR- Enzyms

Das Substratspektrum der N-Acetylaminosäureracemase aus Amycolatopsis orientalis subsp. lurida wurde mit dem unten beschriebenen Enzymassay getestet.

Der Assay setzte sich wie folgt zusammen:

| | |
|---|---|
| Puffer Tris/HCl | 50 mM (pH 8,0) |
| Substrat | 25 mM |
| Cobaltchlorid | 6 mM |
| AAR | ca. 150 µg gereinigtes Protein |
| Endvolumen | 1 ml |

Im Assay wurden enantiomerenreine Aminosäure-Derivate eingesetzt und die Bildung des entsprechenden Racemats im Polarimeter (Perkin-Elmer 241) verfolgt. Die Inkubation erfolgte bei 30°C (heizbare Küvette) für 3 bis 12 Stunden.
Die Messungen erfolgten bei einer Wellenlänge von λ = 365 nm.

**Tabelle 1: Auflistung der getesteten Substrate und der entsprechenden spezifischen Aktivität der AAR.**

| **Substrat** | **Spezifische Aktivität** |
|---|---|
| *N*-Carbamoyl-D-Met | 155 mU/mg |
| *N*-Carbamoyl-D-Phe | 20 mU/mg |
| | |
| *N*-Carbamoyl-L-Abs | 15 mU/mg |
| *N*-Carbamoyl-L-Leu | 20 mU/mg |
| *N*-Carbamoyl-L-Met | 118 mU/mg |
| *N*-Carbamoyl-L-Tyr | 62 mU/mg |
| *N*-Carbamoyl-L-Val | 20 mU/mg |

Die N-Acylaminosäureracemase aus A. TS-1-60 besitzt mit N-Carbamoyl-D-Met als Substrat eine Aktivität von 100 mU/mg. Damit ist diese spezifische Aktivität um 35% niedriger, als die der Racemase aus A. orientalis subsp. lurida.

## Patentansprüche

1. Verwendung von N-Acetylaminosäureracemasen (AAR) aus Amycolatopsis orientalis subspecies lurida in einem Verfahren zur Racemisierung von N-Carbamoylaminosäuren.

2. Verwendung nach Anspruch 1 in einem Prozeß zur Herstellung von enantiomer angereicherten Aminosäuren oder deren Derivaten ausgehend von Hydantoinen oder N-Carbamoylaminosäuren.

3. Verwendung nach einem der vorhergehenden Ansprüche,
**dadurch gekennzeichnet, daß**
man den Prozeß in einem Enzym-Membran-Reaktor durchführt.

## Claims

1. Use of N-acetyl amino acid racemases (AARs) from Amycolatopsis orientalis subspecies lurida in a method of racemizing N-carbamoyl amino acids.

2. Use according to Claim 1 in a process for producing enantiomerically enriched amino acids or derivatives thereof proceeding from hydantoins or N-carbamoyl amino acids.

3. Use according to either of the preceding claims, **characterized in that** the process is conducted in an enzyme-membrane reactor.

## Revendications

1. Utilisation de racémases d'acides N-acétylaminés (AAR) d'Amycolatopsis orientalis de la sous-espèce lurida dans un procédé de racémisation d'acides N-carbamoylaminés.

2. Utilisation suivant la revendication 1 dans un procédé de préparation d'acides aminés énantiomériquement enrichis ou de leurs dérivés à partir d'hydantoïnes ou d'acides N-carbamoylaminés.

3. Utilisation suivant l'une quelconque des revendications précédentes, **caractérisée en ce que** l'on entreprend le procédé dans un réacteur enzymatique à membrane.
